# EUROPÄISCHE PATENTANMELDUNG

(11) **EP 1 122 246 A1**
(43) Veröffentlichungstag der Anmeldung: **08.08.2001**
(21) Anmeldenummer: 00102542.8
(22) Anmeldetag: 07.02.2000
(51) Int. Cl.: C07D 301/12, C07D 301/32

(54) **Verfahren zur Epoxidierung von Olefinen**

(71) Anmelder: Degussa AG, 40474 Düsseldorf (DE)
(72) Erfinder: Hofen, Willi, 63517 Rodenbach (DE); Thiele, Georg, Dr., 63452 Hanau (DE)
(74) Vertreter: Sternagel, Fleischer, Godemeyer & Partner Patentanwälte

(57) **Zusammenfassung**

Die vorliegende Erfindung betrifft ein Verfahren zur katalytischen Epoxidierung von Olefinen, bei dem in einer Reaktionsstufe das Olefin mit wäßrigem Wasserstoffperoxid in einem organischen mit Wasser mischbaren Lösungsmittel in Gegenwart eines Titansilicalitkatalysators umgesetzt wird, wobei ein Abgasstrom (5) entsteht, der Olefinoxid, nicht umgesetztes Olefin und Sauerstoff enthält und dieser Abgasstrom (5) in einer Absorptionseinheit mit dem gleichen, wie in der Reaktionsstufe verwendeten, Lösungsmittel in Kontakt gebracht wird und aus der Absorptionseinheit ein mit Olefin und Olefinoxid beladener Lösungsmittelstrom (17) entnommen wird und ein sauerstoffhaltiger Abgasstrom (16) ausgeschleust wird.

## Beschreibung

Die vorliegende Erfindung betrifft ein Verfahren zur Epoxidierung von Olefinen, bei dem der aus dem Reaktor austretende Abgasstrom weiter aufgearbeitet wird.

### Stand der Technik

Aus EP 100 118 ist bekannt, daß sich Propen mit Wasserstoffperoxid zu Propenoxid umsetzen läßt, wenn als Katalysator Titansilicalit eingesetzt wird. Als Nebenreaktion tritt am Titansilicalitkatalysator stets in geringem Umfang die Zersetzung von Wasserstoffperoxid unter Bildung von molekularem Sauerstoff auf. Um den Epoxidierungsprozeß im technischen Maßstab sicher betreiben zu können, muß der gebildete Sauerstoff aus dem Reaktionssystem entfernt werden. Dies geschieht am einfachsten durch Ausschleusung mit einem Propenabgasstrom. Ein entsprechendes Verfahren ist aus EP 659 473 bekannt. Das Verfahren hat jedoch den Nachteil, daß zusammen mit dem Sauerstoff erhebliche Mengen an Propen und Propenoxid verloren gehen.

Aufgabe der vorliegenden Erfindung ist es daher, ein Verfahren zur Epoxidierung von Olefinen bereitzustellen, mit dem höhere Produktausbeuten erzielt werden können.

### Gegenstand der Erfindung

Diese Aufgabe wird durch ein Verfahren zur katalytischen Epoxidierung von Olefinen, bei dem in einer Reaktionsstufe das Olefin mit wäßrigem Wasserstoffperoxid in einem organischen mit Wasser mischbaren Lösungsmittel in Gegenwart eines Titansilicalitkatalysators umgesetzt wird, wobei ein Abgasstrom entsteht, der Olefinoxid, nicht umgesetztes Olefin und Sauerstoff enthält und dieser Abgasstrom in einer Absorptionseinheit mit dem gleichen, wie in der Reaktionsstufe verwendeten, Lösungsmittel in Kontakt gebracht wird und aus der Absorptionseinheit ein mit Olefin und Olefinoxid beladener Lösungsmittelstrom entnommen wird und ein sauerstoffhaltiger Abgasstrom ausgeschleust wird.

### Beschreibung der Erfindung:

Es wurde nun gefunden, daß sich bei der Epoxidierung von Olefin mit Wasserstoffperoxid und einem Titansilicalitkatalysator die Verluste an Olefin und Olefinoxid bei der Ausschleusung des sauerstoffhaltigen Abgasstroms in einfacher Weise verringern lassen, indem der größte Teil des darin enthaltenen Olefinoxids, Olefins und gegebenenfalls des korrespondierenden Alkans mit dem für die Epoxidierung verwendeten Lösungsmittel absorbiert wird, der Sauerstoff ausgeschleust wird und der mit Olefinoxid und Olefin beladene Lösungsmittelstrom entweder in die Reaktionsstufe zurückgeführt wird oder einer der Reaktionsstufe nachgeschalteten Aufarbeitungsstufe zugeführt wird.

In einer bevorzugten Ausführungsform wird zusätzlich ein Inertgasstrom in die Absorptionseinheit eingeleitet wird, wobei das Inertgas zusammen mit dem Sauerstoff in dem Abgasstrom die Absorptionseinheit verläßt. Die Menge an eingeleitetem Inertgas wird dabei vorzugsweise in Abhängigkeit der Menge und Zusammensetzung des aus der Reaktionsstufe austretenden Abgasstroms so gewählt, daß der die Absorptionseinheit verlassende Abgasstrom eine nicht mehr zündfähige Zusammensetzung aufweist. Diese Ausführungsform hat den Vorteil, daß in sehr einfacher Weise, selbst bei sich ändernden Produktströmen im Gesamtverfahren, die Zusammensetzung der Gasphase in der Absorptionseinheit immer so eingestellt werden kann, daß weder eine zündfähige Mischung innerhalb der Absorptionseinheit entstehen kann, noch diese als Abgasstrom verlassen kann.

Als Inertgas eignen sich alle Gase, die sich in dem zur Epoxidierung verwendeten Lösungsmittel nur in geringem Maße lösen, mit Wasserstoffperoxid und Olefinoxid unter den Bedingungen der Epoxidierung nicht reagieren und mit Sauerstoff keine explosionsfähigen Gemische bilden. Bevorzugt wird als Inertgas Stickstoff oder ein durch Verbrennung einer Methan-Luft-Mischung erhaltenes Inertgas eingesetzt.

Als Lösungsmittel geeignet sind alle Lösungsmittel, die unter den gewählten Reaktionsbedingungen nicht oder nur in geringem Maß durch Wasserstoffperoxid oxidiert werden und sich mit mehr als 10 Gew.-% in Wasser lösen. Bevorzugt werden Lösungsmittel, die mit Wasser unbegrenzt mischbar sind. Geeignete Lösungsmittel sind Alkohole wie z.B. Methanol, Ethanol oder tert-Butanol; Glykole wie z.B. Ethylenglykol, 1,2-Propandiol oder 1,3-Propandiol; cyclische Ether wie z.B. Tetrahydrofuran, Dioxan oder Propenoxid; Glykolether wie z.B. Ethylenglykolmonomethylether, Ethylenglykolmonoethylether, Ethylenglykolmonobutylether oder die Propylenglykolmonomethylether und Ketone wie z.B. Aceton oder 2-Butanon. Besonders bevorzugt wird Methanol als Lösungsmittel eingesetzt. Die Absorption wird bei einem Gesamtdruck im Bereich von 1 bis 25 bar durchgeführt, vorzugsweise bei dem gleichen Druck wie die Epoxidierungsreaktion, bei der das sauerstoffhaltige Abgas anfällt. Die Absorption kann bei Temperaturen zwischen dem Schmelzpunkt des Lösungsmittels und 100°C durchgeführt werden, vorzugsweise im Bereich von 0 bis 60°C.

In einer besonders bevorzugten Ausführungsform der vorliegenden Erfindung wird der Inertgasstrom und der Abgasstrom im Gegenstrom zum Lösungsmittel geführt. Für diese Ausführungsform eignet sich insbesondere eine Kolonne mit inerten Füllkörpern oder Einbauten als Absorptionseinheit, wobei der olefin- und olefinoxidhaltige Abgasstrom und der Inertgasstrom in den Sumpf der Kolonne eingespeist werden, das Lösungsmittel am Kopf der Kolonne aufgegeben wird, der Abgasstrom am Kopf der Kolonne ausgeschleust wird, und der mit Olefin und Olefinoxid beladene Lösungsmittelstrom aus dem Sumpf der Kolonne entnommen wird.

Das erfindungsgemäße Verfahren eignet sich zur Epoxidierung von Olefinen mit 2 bis 6 Kohlenstoffatomen. Am meisten bevorzugt ist die Epoxidierung von Propen zu Propenoxid. Daher wird im folgenden das erfindungsgemäße Verfahren am Beispiel der Epoxidierung von Propen näher erläutert.

Fig. 1 zeigt eine Ausführungsform der Erfindung; bei der die Absorptionsstufe so in das Epoxidierungsverfahren eingebunden ist, daß der in der Absorption mit Propen und Propenoxid beladene Lösungsmittelstrom in die Epoxidierungsreaktion geführt wird.

Fig. 2 zeigt eine alternative Ausführungsform der vorliegenden Erfindung, bei der der mit Propen, Propan und Propenoxid beladene Lösungsmittelstrom nicht in die Epoxidierungsstufe, sondern in die Aufarbeitungsstufe geführt wird.

Fig. 3 zeigt eine für das erfindungsgemäße Verfahren geeignete Absorptionseinheit.

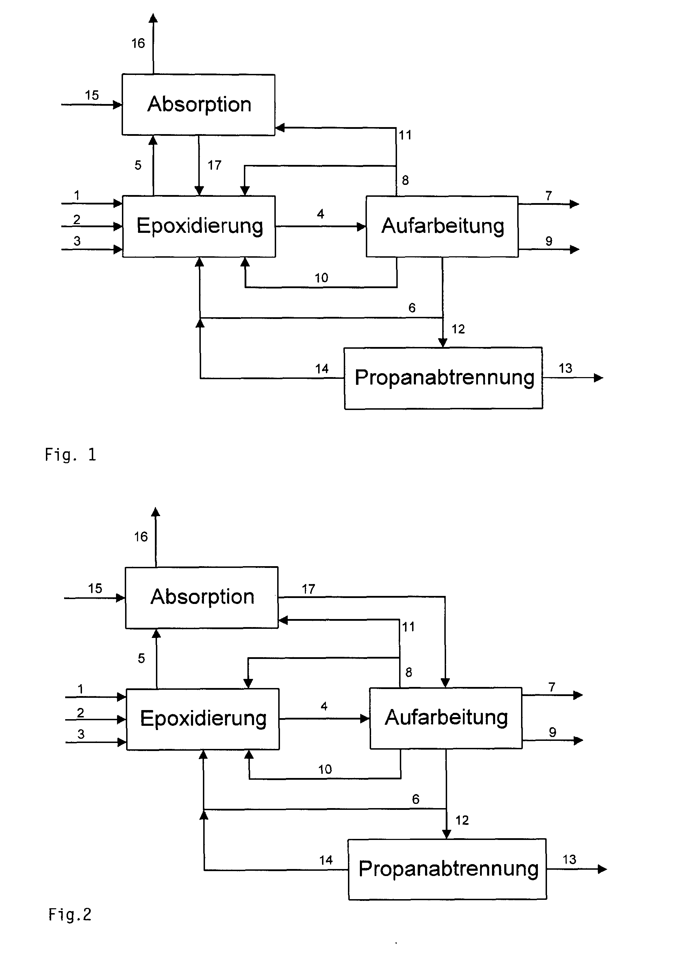

Gemäß Fig. 1 bzw. Fig. 2 werden in die Epoxidierungsstufe Propen mit Strom 1, Wasserstoffperoxid mit Strom 2 und das Lösungsmittel mit Strom 3 eingespeist, wobei Strom 3 der Ergänzung von Lösungsmittelverlusten im Prozeß dient. Die Epoxidierungsstufe verläßt eine flüssige Reaktionsmischung mit Strom 4 und ein sauerstoffhaltiges Abgas mit Strom 5. Die flüssige Reaktionsmischung von Strom 4 wird in der Aufarbeitung aufgetrennt in Strom 6, der im wesentlichen aus Propen und Propan besteht, Strom 7, der im wesentlichen aus Propenoxid besteht, Strom 8, der im wesentlichen aus dem Lösungsmittel besteht und Strom 9, der im wesentlichen aus Wasser und hochsiedenden Nebenprodukten besteht. Bei der Verwendung eines Suspensionskatalysators wird in der Aufarbeitung außerdem mit Strom 10 der Katalysator zurückgewonnen, der in die Epoxidierung zurückgeführt wird, wobei optional zuvor ein Teil des Katalysators oder der ganze Katalysator einem Regenerierschritt unterworfen wird. Bei der Verwendung eines verformten Katalysators, der in der Epoxidierungsstufe zurückgehalten wird, entfällt Strom 10. Das zurückgewonnene Lösungsmittel aus Strom 8 wird ganz oder teilweise mit Strom 11 der Absorptionsstufe zugeführt. Von dem Propenstrom 6, der in die Epoxidierung zurückgeführt wird, wird ein Teilstrom 12 der Propanabtrennung zugeführt, aus der ein mit Propan angereicherter Strom 13 ausgeschleust wird, während der von Propan abgereicherte Strom 14 ebenfalls der Epoxidierung zugeführt wird.

In die Absorptionsstufe wird zusätzlich zu dem sauerstoffhaltigen Abgasstrom 5 und dem Lösungsmittelstrom 11 noch ein Inertgasstrom 15 eingespeist. Das Inertgas verläßt die Absorptionsstufe zusammen mit dem Sauerstoff aus der Epoxidierung mit dem Abgasstrom 16.

Bei der in Fig. 1 gezeigten Ausführungsform wird der mit Propen, Propan und Propenoxid beladene Lösungsmittelstrom 17 in die Epoxidierungsstufe zurückgeführt und der Lösungsmittelstrom 8 so aufgeteilt, daß vorzugsweise mehr als 30% und besonders bevorzugt mehr als 80% mit Strom 11 in die Absorptionsstufe geführt werden.

Bei der in Fig. 2 gezeigten Ausführungsform wird der mit Propen, Propan und Propenoxid beladene Lösungsmittelstrom 17 in die Aufarbeitungsstufe geführt wird und der Lösungsmittelstrom 8 so aufgeteilt, daß vorzugsweise weniger als 50% und besonders bevorzugt weniger als 30% mit Strom 11 in die Absorptionsstufe geführt werden.

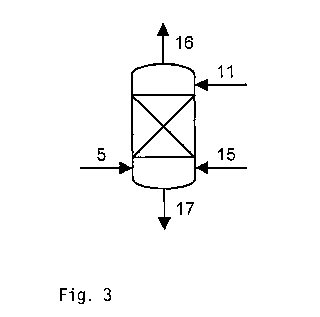

In der Absorptionsstufe werden der Abgasstrom 5 und der Lösungsmittelstrom 11 im Gegenstrom geführt. Die Absorptionsstufe wird bevorzugt wie in Fig. 3 gezeigt als Kolonne mit inerten Füllkörpern oder Einbauten ausgeführt und so betrieben, das die Gasströme 5 und 15 in den Sumpf der Kolonne eingespeist und der Abgasstrom 16 am Kopf der Kolonne entnommen wird, während das Lösungsmittel mit Strom 11 am Kopf der Kolonne aufgegeben wird und der beladene Flüssigkeitsstrom 17 im Sumpf der Kolonne abgezogen wird.

Die Menge an Inertgas wird so gewählt, daß der Abgasstrom 16, der neben dem Inertgas noch Sauerstoff, sowie geringe Mengen an Propen und Lösungsmittel enthält, eine nicht mehr zündfähige Zusammensetzung aufweist.

In der Epoxidierungsstufe kann der wasserstoffperoxidhaltige Strom 2 mit einem lösungsmittelhaltigen Strom (Strom 8 und/oder 17 in der Ausführungsform nach Fig. 1 bzw. Strom 8 in der Ausführungsform nach Fig. 2) vermischt werden, bevor er dem Epoxidierungsreaktor zugeführt wird. Ebenso können die propenhaltigen Ströme 1 und 6 vermischt werden, bevor sie in den Epoxidierungsreaktor eingespeist werden.

Das erfindungsgemäße Verfahren hat den Vorteil, daß mit dem sauerstoffhaltigen Abgasstrom nur geringe Mengen an Propen und Propenoxid verloren gehen, und daß zur Rückgewinnung des Propens und Propenoxids aus dem Abgasstrom keine zusätzlichen Hilfsstoffe benötigt werden, da das für die Absorption verwendete Lösungsmittel zusammen mit dem in der Epoxidierungsreaktion eingesetzten Lösungsmittel im Kreislauf geführt werden kann.

## Patentansprüche

1. Verfahren zur katalytischen Epoxidierung von Olefinen, bei dem in einer Reaktionsstufe das Olefin mit wäßrigem Wasserstoffperoxid in einem organischen mit Wasser mischbaren Lösungsmittel in Gegenwart eines Titansilicalitkatalysators umgesetzt wird, wobei ein Abgasstrom (5) entsteht, der Olefinoxid, nicht umgesetztes Olefin und Sauerstoff enthält,
dadurch gekennzeichnet, daß
dieser Abgasstrom (5) in einer Absorptionseinheit mit dem gleichen, wie in der Reaktionsstufe verwendeten, Lösungsmittel in Kontakt gebracht wird und aus der Absorptionseinheit ein mit Olefin und Olefinoxid beladener Lösungsmittelstrom (17) entnommen wird und ein sauerstoffhaltiger Abgasstrom (16) ausgeschleust wird.

2. Verfahren nach Anspruch 1,
dadurch gekennzeichnet, daß
zusätzlich ein Inertgasstrom (15) in die Absorptionseinheit eingeleitet wird, wobei das Inertgas zusammen mit dem Sauerstoff in dem Abgasstrom (16) die Absorptionseinheit verläßt.

3. Verfahren nach Anspruch 2,
dadurch gekennzeichnet, daß
die Menge an eingeleitetem Inertgas in Abhängigkeit der Menge und Zusammensetzung des aus der Reaktionsstufe austretenden Abgasstroms (5) so gewählt wird, daß der die Absorptionseinheit verlassende Abgasstrom (16) eine nicht mehr zündfähige Zusammensetzung aufweist.

4. Verfahren nach einem der Ansprüche 2 und 3
dadurch gekennzeichnet, daß
das Inertgas aus einem Gas, das sich in dem zur Epoxidierung verwendeten Lösungsmittel nur in geringem Maße löst, mit Wasserstoffperoxid und dem Olefinoxid unter den Bedingungen der Epoxidierung nicht reagiert und mit Sauerstoff keine explosionsfähigen Gemische bildet, vorzugsweise aus Stickstoff oder einem durch Verbrennung einer Methan-Luft-Mischung erhaltenen Gas ausgewählt ist.

5. Verfahren nach einem der Ansprüche 2-4,
dadurch gekennzeichnet, daß
der Inertgasstrom (15) und der Abgasstrom (5) im Gegenstrom zum Lösungsmittel geführt wird.

6. Verfahren nach einem der vorstehenden Ansprüche,
dadurch gekennzeichnet, daß
als Absorptionseinheit eine Kolonne mit inerten Füllkörpern oder Einbauten verwendet wird und die Gasströme (5, 15) in den Sumpf der Kolonne eingespeist werden, das Lösungsmittel als Lösungsmittelstrom (11) am Kopf der Kolonne aufgegeben wird, der Abgasstrom (16) am Kopf der Kolonne ausgeschleust wird, und der mit Olefin und Olefinoxid beladene Lösungsmittelstrom (17) aus dem Sumpf der Kolonne entnommen wird.

7. Verfahren nach einem der vorstehenden Ansprüche,
dadurch gekennzeichnet, daß
der mit Olefin und Olefinoxid beladene Lösungsmittelstrom (17) entweder in die Reaktionsstufe zurückgeführt oder einer der Reaktionsstufe nachgeschalteten Aufarbeitungsstufe zugeführt wird.

8. Verfahren nach einem der vorstehenden Ansprüche,
dadurch gekennzeichnet, daß
der flüssige Produktstrom (4) aus der Reaktionsstufe aufgearbeitet wird und das dabei anfallende zurückgewonnene Lösungsmittel teilweise in die Absorptionseinheit und teilweise in die Reaktionsstufe zurückgeführt wird.

9. Verfahren nach einem der vorstehenden Ansprüche,
dadurch gekennzeichnet, daß
das Olefin ein Olefin mit 2-6 Kohlenstoffatomen, vorzugsweise Propen ist.

10. Verfahren nach einem der vorstehenden Ansprüche,
dadurch gekennzeichnet, daß
das Lösungsmittel ausgewählt ist aus Alkoholen, Glykolen , cyclische Ethern, Glykolethern und Ketonen und vorzugsweise Methanol ist.
